# EUROPEAN PATENT SPECIFICATION

(11) **EP 4 183 345 B1**
(45) Date of publication and mention of the grant of the patent: **01.10.2025**
(21) Application number: 21857789.8
(22) Date of filing: 23.08.2021
(51) Int. Cl.: A61B 6/00, A61B 10/02, A61B 34/30, A61B 6/04

(54) **BREAST X-RAY IMAGING APPARATUS, AND METHOD AND SYSTEM FOR CORRECTING AND VERIFYING BREAST BIOPSY POSITIONING DEVICE**
BRUST-RÖNTGENBILDGEBUNGSVORRICHTUNG SOWIE VERFAHREN UND SYSTEM ZUR KORREKTUR UND ÜBERPRÜFUNG EINER BRUST-BIOPSIE-POSITIONIERUNGSVORRICHTUNG
APPAREIL D'IMAGERIE PAR RAYONS X DU SEIN, ET PROCÉDÉ ET SYSTÈME DE CORRECTION ET DE VÉRIFICATION DU DISPOSITIF DE POSITIONNEMENT DE BIOPSIE DU SEIN

(30) Priority: 21.08.2020 CN 202010850716; 20.10.2020 CN 202011123415
(43) Date of publication of application: 24.05.2023
(73) Proprietor: Shanghai United Imaging Healthcare Co., Ltd., Shanghai 201807 (CN)
(72) Inventor: LIANG, Kan, Shanghai 201807 (CN); XIA, Angran, Shanghai 201807 (CN); CHU, Dongwei, Shanghai 201807 (CN)
(74) Representative: Wang, Bo
(86) International application number: PCT/CN2021/114108
(87) International publication number: WO 2022/037702

(56) References cited:
- CN-A- 102 327 122
- CN-A- 102 481 146
- CN-A- 105 726 133
- CN-A- 108 335 326
- CN-A- 109 009 348
- CN-A- 111 839 567
- CN-A- 112 022 229
- CN-U- 204 133 565
- DE-A1- 102016 205 210
- US-A1- 2002 035 864
- US-A1- 2010 261 997
- US-A1- 2018 271 458
- US-A1- 2018 271 458
- US-A1- 2020 214 591
- ZELIKMAN M I ET AL: "Experience in the Use of a Test Phantom for Checking Biopsy Needle Position in Stereotactic Mammography", BIOMEDICAL ENGINEERING, KLUWER ACADEMIC PUBLISHERS-CONSULTANTS BUREAU, NE, vol. 39, no. 5, 1 September 2005 (2005-09-01), pages 248 - 250, XP019218439, ISSN: 1573-8256, DOI: 10.1007/S10527-006-0012-Z

## Description

### CROSS-REFERENCE TO RELATED APPLICATIONS

This application claims priority to Chinese Patent Application No. 202010850716.6, filed on August 21, 2020, and Chinese Patent Application No. 202011123415.X, filed on October 20, 2020.

### TECHNICAL FIELD

The present disclosure generally relates to medical devices and imaging technology, and more particularly, relates to breast X-ray imaging apparatus, methods and systems for correcting and verifying a breast biopsy positioning device.

### BACKGROUND

Diagnosis of breast diseases usually uses breast devices to perform the disease screening and the micro-invasive puncture sampling. During the puncture sampling, a ray tube and a detector of a breast device rotate relative to a gantry to acquire imaging images of a lesion at different angles. For example, an image of a scout frame and images of stereo pair frames obtained before the puncture, an image of a pre-fire frame obtained during the puncture, and an image of a post-fire frame obtained after the puncture are provided to a physician for reference to determine a position of the lesion.

A needle holder of a traditional breast device is mounted on a gantry of the breast device and arranged between a ray tube and a detector. To avoid the needle holder from being captured (i.e., irradiated) during imaging, the needle holder needs to be moved to the farthest end to avoid the needle holder from entering an irradiated region during capturing the image of the scout frame. However, it is impossible to avoid the needle holder from being captured during image capture of stereo pair frames, the image of the pre-fire frame, and the image of the post-fire frame, so that part of the captured image is blocked by the needle holder which affects the diagnosis of the physician.

### SUMMARY

According to a first aspect of the present disclosure, a breast X-ray imaging apparatus is provided. The breast X-ray imaging apparatus includes a detection mechanism and a needle holder. The detection mechanism includes a ray tube and a detector that is arranged opposite to the ray tube. An irradiated region is arranged between the ray tube and the detector. The needle holder is configured to move relative to the detection mechanism, and the needle holder is arranged outside the irradiated region.

In one embodiment, the breast X-ray imaging apparatus includes a mechanical arm. The needle holder is mounted on the mechanical arm, and the mechanical arm is configured to drive the needle holder to move relative to the detection mechanism.

The mechanical arm is configured to drive the needle holder to move, so that the movement of the needle holder may be more flexible, thereby facilitating the needle holder to be inserted at an optimal inserting angle during puncture to reduce a depth of a wound of a patient. In addition, the needle holder is mounted on the mechanical arm to avoid repeated disassemble and assemble, which not only reduces an error caused by the installation of the needle holder, but also further reduces a calibration duration of the needle holder, thereby improving diagnosis efficiency.

In one embodiment, the detection mechanism includes a gantry. The ray tube and the detector may be mounted on the gantry. The mechanical arm is arranged independently from the detection mechanism. A mounting position of the mechanical arm is relatively fixed to a position of the gantry.

The mechanical arm may be fixed on a ward wall, ceiling, floor, or other positions that are independent from a breast device, thereby increasing a movement flexibility of the mechanical arm relative to the breast device and reducing a count of position calibrations of the mechanical arm. The mechanical arm may not need to be calibrated before usage per time. That is, the mechanical arm may be calibrated after a certain period of usage, and the time interval of the calibration period may be increased to 1 to 3 months.

In one embodiment, the detection mechanism may further include a compression plate and a compression platform. The compression plate and the compression platform may be mounted on the gantry. The compression plate and the compression platform may be arranged between the ray tube and the detector, and the compression plate may be arranged opposite to the compression platform. The breast X-ray imaging apparatus further may include a puncture needle. The puncture needle may be mounted on the needle holder, and the mechanical arm may be configured to drive the puncture needle to be inserted along a tilt direction relative to the compression plate.

Since the breast is a flexible tissue, the mutual cooperation between the compression plate and the compression platform may enable the breast to be deformed into a relatively thin and uniform shape, thereby facilitating an acquisition of a high quality image. In addition, for a traditional breast device, a puncture needle may be merely inserted along a vertical direction or a horizontal direction. In the present disclosure, the needle holder and the puncture may move with the mechanical arm, thereby greatly increasing an inserting angle range and flexibility of the puncture needle. The puncture needle may be inserted along the vertical direction, the horizontal direction, or the tilt direction, thereby reducing the depth of the wound of the patient.

In one embodiment, the detection mechanism may further include a rotating support. The rotating support may be rotatably mounted on the gantry. The ray tube and the detector may be mounted on two ends of an opening of the rotating support, and the rotating support may be configured to rotate relative to the compression plate and the compression platform.

A compression plate and a compression platform of a traditional breast device may usually rotate with a gantry. During an examination of a breast, after a rotation of a rotating support, the breast of the patient may need to be re-positioned, which extends examination time. In the present disclosure, the rotating support may rotate relative to the compression plate and the compression platform. During the examination, the breast of the patient may not need to be re-positioned, and images from different angles may be captured based on the rotation of the rotating support.

In one embodiment, the mechanical arm may include at least two arm joints. The at least two arm joints may be rotatably connected. An end of one arm joint of the at least two arm joints may be relatively fixed to the position of the gantry, and the needle holder may be mounted on an end of another one arm joint of the at least two arm joints.

The flexibility of the mechanical arm's usage may be improved, which may be convenient for the physician to adjust the needle holder and the puncture needle to an appropriate inserting angle in order to improve the diagnosis efficiency.

In one embodiment, at least one of the at least two arm joints may be a retractable arm joint.

The flexibility of the mechanical arm's usage may be further improved.

In one embodiment, the breast X-ray imaging apparatus includes a host. The host is communicatively connected with the mechanical arm and configured to control a movement of the mechanical arm.

The host is configured to control a movement of the mechanical arm, which is convenient for an operation of the physician. There is no need to provide an additional device configured to control the movement of the mechanical arm, which reduce an occupied area of the breast X-ray imaging apparatus.

In one embodiment, the breast X-ray imaging apparatus may further include a photographing unit. The photographing unit is communicatively connected with the host, and the needle holder is arranged outside the irradiated region based on a visual navigation of the photographing unit.

The photographing unit may help the physician to observe a real time condition of the breast during puncture to avoid uncomfortable of the patient. The photographing unit may also cooperate with the host to guide the mechanical arm to move and avoid obstacles through the visual navigation, and the mechanical arm may drive the needle holder and the puncture needle to move to an optimal inserting position and perform the puncture operation.

In one embodiment, the host may be further configured to control a movement of the photographing unit, and a mounting position of the photographing unit may be relatively fixed to the position of the gantry.

The position of the photographing unit may be relatively fixed, thereby increasing the time interval of the calibration period and making a process of the visual navigation more accurate.

For the breast X-ray imaging apparatus provided in the present disclosure, the needle holder may move relative to the breast. The needle holder and the breast device may be arranged outside the irradiated region when the needle holder and the breast device are in a capture state before, during, and after puncture, that is, when the image of the scout frame, the images of stereo pair frames, the image of the pre-fire frame, and the image of the post-fire frame are captured. On the one hand, the breast X-ray imaging apparatus provided in the present disclosure may optimize the image captured by the breast device to avoid an appearance of the needle holder in the image captured before and after puncture, thereby achieving a better diagnosis of the position of the lesion and preventing the lesion from being blocked by the needle holder during imaging. On the other hand, an operation efficiency of the physician may be improved, and the needle holder may not need to be moved to the farthest end when capturing the image of the scout frame, thereby reducing a diagnosis period and avoiding a repeated capture caused by the physician forgetting to move the needle holder to the farthest end before the image of the scout frame is captured.

According to a second aspect of the present disclosure, a method for correcting and verifying a breast biopsy positioning device is provided. The breast biopsy positioning device may be mounted on a breast X-ray imaging apparatus, and the breast biopsy positioning device may be connected with a needle holder. The method may include in response to a first driving instruction, driving the needle holder to move a needle tip of a puncture needle mounted on the needle holder to a first preset position. The method may also include in response to a movement termination signal, collecting a first pair of stereo positioning images of a region relative to the needle tip of the puncture needle, acquiring, based on the first pair of stereo positioning images, coordinates of a real position of the needle tip and coordinates of the first preset position, and acquiring a coordinate difference between the first preset position and the real position. The method may further include in response to the acquisition of the coordinate difference, correcting the breast biopsy positioning device based on the coordinate difference.

In one embodiment, the acquiring coordinates of a real position of the needle tip and coordinates of the first preset position based on the first pair of stereo positioning images may include for each image of the first pair of stereo positioning images, identifying coordinates of a first preset label, and determining the coordinates of the real position and the coordinates of the first preset position based on the coordinates of the first preset label. The each image of the first pair of stereo positioning images may include the first preset label of a same position. The correcting the breast biopsy positioning device based on the coordinate difference may include correcting the breast biopsy positioning device based on a coordinate difference corresponding to the each image of the first pair of stereo positioning images.

In one embodiment, there may be a plurality of first preset positions, and the method may further include moving the needle tip of the puncture needle to a next first preset position after acquiring the coordinate difference at a current first preset position, acquiring a correction compensation value of the breast biopsy positioning device based on coordinate differences obtained at the plurality of first preset positions, and correcting the breast biopsy positioning device based on the correction compensation value.

According to a third aspect of the present disclosure, a system for correcting and verifying a breast biopsy positioning device is provided. The breast biopsy positioning device may be mounted on a breast X-ray imaging apparatus, and the breast biopsy positioning device may be connected with a needle holder. The system may include a first needle holder driving unit, in response to a first driving instruction, configured to drive the needle holder to move a needle tip of a puncture needle mounted on the needle holder to a first preset position. The system may also include a first position processing unit, in response to a movement termination signal, configured to collect a first pair of stereo positioning images of a region relative to the needle tip of the puncture needle, acquire coordinates of a real position of the needle tip and coordinates of the first preset position based on the first pair of stereo positioning images, and acquire a coordinate difference between the first preset position and the real position. The system may further include a positioning correction unit, in response to the acquisition of the coordinate difference, configured to correct the breast biopsy positioning device based on the coordinate difference.

According to a fourth aspect of the present disclosure, another method for correcting and verifying a breast biopsy positioning device is provided. The breast biopsy positioning device may be mounted on a breast X-ray imaging apparatus, and the breast biopsy positioning device may be connected with a needle holder. The method may include installing a phantom under the breast biopsy positioning device, and in response to a second driving instruction, compressing the phantom through a compression plate of the detection mechanism. The method may also include in response to a compression termination signal, collecting a second pair of stereo positioning images of the phantom, and acquiring coordinates of a correction position based on the second pair of stereo positioning images. The method may also include in response to the acquisition of the coordinates of the correction position, driving the needle holder to move a needle tip of a puncture needle mounted on the needle holder to the correction position. The method may also include in response to a movement termination signal, collecting a third pair of stereo positioning images of the phantom, acquiring coordinates of a position of the needle tip of the puncture needle based on the third pair of stereo positioning images, and acquiring a coordinate difference based on the coordinates of the correction position and the coordinates of the position of the needle tip of the puncture needle. The method may further include in response to the acquisition of the coordinate difference, verifying the breast biopsy positioning device based on the coordinate difference.

In one embodiment, the acquiring coordinates of a correction position based on the second pair of stereo positioning images may include for each image of the second pair of stereo positioning images, identifying coordinates of a second preset label, and determining the coordinates of the correction position based on the coordinates of the second preset label. The each image of the second pair of stereo positioning images may include the second preset label of a same position. The acquiring coordinates of a position of the needle tip of the puncture needle based on the third pair of stereo positioning images may include for each image of the third pair of stereo positioning images, identifying the coordinates of the second preset label, and determining the coordinates of the position of the needle tip of the puncture needle based on the coordinates of the second preset label. The each image of the third pair of stereo positioning images may include the second preset label of a same position.

In one embodiment, there may be a plurality of correction positions, and the method further may include moving the needle tip of the puncture needle to a next correction position after verifying the breast biopsy positioning device at a current correction position, and acquiring a target verification result of the breast biopsy positioning device based on verification results obtained at the plurality of correction positions.

According to a fifth aspect of the present disclosure, another system for correcting and verifying a breast biopsy positioning device is provided. The breast biopsy positioning device may be mounted on a breast X-ray imaging apparatus, and the breast biopsy positioning device may be connected with a needle holder. The system may include a correcting and verifying preparation unit configured to install a phantom under the breast biopsy positioning device, and in response to a second driving instruction, compress the phantom through a compression plate of the detection mechanism. The system may also include a correction position determination unit, in response to a compression termination signal, configured to collect a second pair of stereo positioning images of the phantom, and acquire coordinates of a correction position based on the second pair of stereo positioning images. The system may also include a second needle holder driving unit, in response to the acquisition of the coordinates of the correction position, configured to drive the needle holder to move the needle tip of the puncture needle mounted on the needle holder to the correction position. The system may also include a second position processing unit, in response to a movement termination signal, configured to collect a third pair of stereo positioning images of the phantom, acquire coordinates of the needle tip of the puncture needle based on the third pair of stereo positioning images, and acquire a coordinate difference based on the correction position and the needle tip of the puncture needle. The system may further include a positioning verification unit, in response to the acquisition of the coordinate difference, configured to verify the breast biopsy positioning device based on the coordinate difference.

According to a sixth aspect of the present disclosure, a non-transitory computer readable medium is provided. The non-transitory computer readable medium may include a set of instructions. When executed by at least one processor, the set of instructions may direct the at least one processor to effectuate any operation of a method for correcting and verifying a breast biopsy positioning device.

According to a seventh aspect of the present disclosure, an apparatus for correcting and verifying a breast biopsy positioning device is provided. The apparatus may include at least one processor and at least one storage device. The at least one storage device may include a set of instructions. When executed by the at least one processor, the set of instructions may direct the at least one processor to effectuate any operation of a method for correcting and verifying a breast biopsy positioning device.

For the method, system, non-transitory computer readable medium, and apparatus for correcting and verifying a breast biopsy positioning device provided in the present disclosure during an accuracy correction and verification of the breast biopsy positioning device, the needle holder of the breast biopsy positioning device may be driven to move based on different workflows of the correction process and the verification process to move the needle tip of the puncture needle to a preset position, and a pair of stereo positioning images may be collected and identified. The accuracy correction and verification of the breast biopsy positioning device may be achieved by measuring the position of the needle tip and the preset position, thereby reducing human error introduced by personnel operation in determination and identification and improving efficiency of the accuracy correction and verification of the breast biopsy positioning device.

Details about one or more embodiments of the present disclosure may be provided in the following drawings and descriptions to make other features, purposes, and advantages of the present disclosure more concise and easily understood. The invention is set out in the appended set of claims.

### BRIEF DESCRIPTION OF THE DRAWINGS

The drawings illustrated herein may provide a further understanding of the present disclosure and constitute part of the present disclosure. These exemplary embodiments and illustrations of these exemplary embodiments may be used to interpretate the present disclosure, which do not constitute an improper limitation of the present disclosure. In the drawings:
FIG. 1 is a schematic diagram illustrating a structure of a breast X-ray imaging apparatus according to an embodiment of the present disclosure;
FIG. 2 is a schematic diagram illustrating a principle of capturing an image of a scout frame and images of stereo pair frames by an imaging component;
FIG. 3 is a schematic diagram illustrating capturing an image of a pre-fire frame by an imaging component;
FIG. 4 is a schematic diagram illustrating an image of a pre-fire frame captured by an imaging component;
FIG. 5 is a schematic diagram illustrating capturing an image of a post-fire frame by an imaging component;
FIG. 6 is a schematic diagram illustrating an image of a post-fire frame captured by an imaging component;
FIG. 7 is a flowchart illustrating a process for correcting and verifying a breast biopsy positioning device according to an embodiment of the present disclosure;
FIG. 8 is a flowchart illustrating a process for correcting and verifying a breast biopsy positioning device according to another embodiment of the present disclosure;
FIG. 9 is a schematic diagram illustrating a structure of a system for correcting and verifying a breast biopsy positioning device according to an embodiment of the present disclosure;
FIG. 10 is a schematic diagram illustrating a structure of a system for correcting and verifying a breast biopsy positioning device according to another embodiment of the present disclosure; and
FIG. 11 is a schematic diagram illustrating an internal structure of an apparatus for correcting and verifying a breast biopsy positioning device according to an embodiment of the present disclosure.

In the drawings: 100, a breast X-ray imaging apparatus; 10, a breast device; 11, a gantry; 12, a rotating support; 13, an imaging component; 131, a ray tube; 132, a detector; 133, an irradiated region; 14, a compression component; 141, a compression plate; 142, a compression platform; 20, a puncture component; 21, a needle holder; 22, a puncture needle; 30, a mechanical arm; 31, an arm joint; 40, a photographing member; 200, a breast; 210, a lesion.

### DETAILED DESCRIPTION

The technical solutions of the present disclosure are clearly and completely described in combination with the drawings of the embodiments of the present disclosure. Obviously, the embodiments described herein are merely part of the embodiments of the present disclosure, which do not include all embodiments. Based on the embodiments of the present disclosure, all other embodiments obtained by those skilled in the art without a creative work are within a scope of the present disclosure.

It will be understood that when a unit is referred to as being "connected to" or "coupled to" another unit, it may be directly on, connected or coupled to, or communicate with the other unit, or an intervening unit may be present. when a unit is referred to as being "fixed on" another unit, it may be directly fixed on the other unit, or an intervening unit may be present.

Unless otherwise defined, the meaning of technical and/or scientific terms used herein may be the same as the meaning that commonly understood by those skilled in the arts. The terms used herein in the present disclosure are for the purpose of describing particular example embodiments only and is not intended to be limiting. The terms "and/or" used herein include any and/or all combinations of one or more listed items.

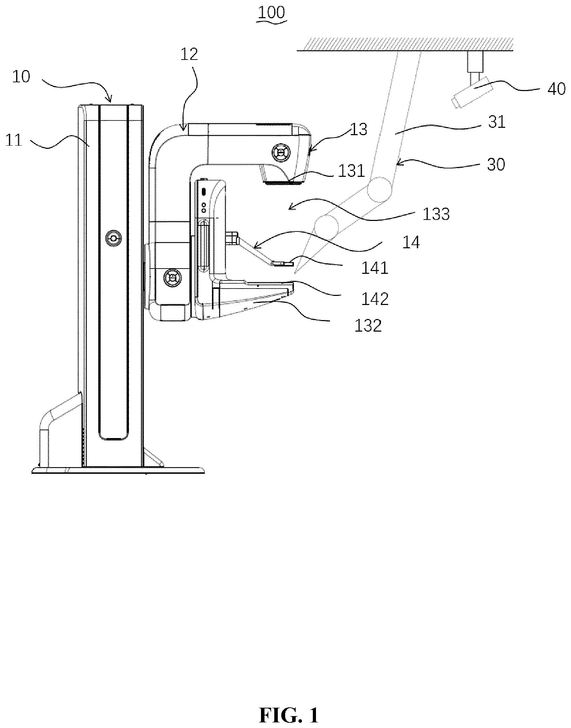

Referring to FIG. 1, FIG. 1 is a schematic diagram illustrating a structure of a breast X-ray imaging apparatus 100 according to an embodiment of the present disclosure.

The present disclosure provides the breast X-ray imaging apparatus 100. The breast X-ray imaging apparatus 100 may be configured to assist a physician in puncture biopsy. In the embodiment, the breast X-ray imaging apparatus 100 may be configured to assist a physician in coarse needle puncture biopsy of the breast. It should be understood that, in another embodiment, the breast X-ray imaging apparatus 100 may be used in other types of puncture biopsy of the breast such as fine needle puncture biopsy of the breast or the like. The breast X-ray imaging apparatus 100 may also be used in other usage situations such as computed tomography (CT) examination, magnetic resonance imaging (MRI) examination, or the like. The following may take a breast device as an example for illustration.

The breast X-ray imaging apparatus 100 may include a breast device 10 and a puncture component 20. The breast device 10 may be configured to shoot a breast 200 and capture an image of the breast 200, so that a physician may locate a lesion 210. The puncture component 20 may be used for puncture sampling of a breast nodule in the breast 200, and the physician may perform a pathological examination on the sampled tissue to determine whether the sampled tissue has a malignant lesion.

The breast device 10 may include a gantry 11, a rotating support 12, an imaging component 13, and a compression plate 14. The rotating support 12 may be rotatably connected to the gantry 11. The imaging component 13 may be mounted on the rotating support 12 and rotate with the rotating support 12 to acquire an X-ray image at different angles. The compression plate 14 may be mounted on the gantry 11. The compression plate 14 may be configured to hold and compress the breast 200 of a patient, so that the breast 200 may be deformed into a relatively thin and uniform shape, thereby facilitating an acquisition of a high quality image.

The gantry 11 may be configured to support the rotating support 12, the imaging component 13, and the compression plate 14. The gantry 11 may be fixed on the ground, which not only prevents the breast device 100 from dumping, but also facilitates a positioning between the breast device 10 and other surgical instruments, thereby increasing a time interval of a position calibration period.

The rotating support 12 may be in an approximately "C" shape. An opening of the rotating support 12 may be arranged on a side relatively far from the gantry 11. The rotating support 12 may rotate relative to the gantry 11, and drive the imaging component 13 mounted on the rotating support 12 to rotate together, so that an angle may be formed between the imaging component 13 and the compression plate 14. The imaging component 13 may acquire images of the breast 200 at different angles to facilitate a determination of a position of the lesion 210 by the physician.

The imaging component 13 may include a ray tube 131 and a detector 132. The ray tube 131 and the detector 132 may be arranged at two ends of the rotating support 12. An irradiated region 133 may be formed between the ray tube 131 and the detector 132. The ray tube 131 may emit X-ray to the detector 132. Due to the penetrability of the X-ray, and different densities and thicknesses of different positions on the breast 200, when the X-ray passes through the different positions on the breast 200, an absorption degree of the X-ray may be different. Therefore, when the X-ray reaches the detector 132, an intensity of the X-ray at the different positions may be different and an depth of an image may also be different, thereby forming an image of the breast 200 of the patient.

The compression component 14 may include a compression plate 141 and a compression platform 142. The compression plate 141 and the compression platform 142 may be mounted on the gantry 11 directly or indirectly. The compression plate 141 and the compression platform 142 may be arranged within the irradiated region 133. The compression plate 141 may be arranged at a side relatively close to the ray tube 131, and the compression platform 142 may be arranged at a side relatively close to the detector 132. The compression plate 141 may be arranged opposite to the compression platform 142. The compression plate 141 may be detachably connected to the gantry 11, and different types of compression plates 141 may be replaced based on different examination requirements of a patient, so that the breast 200 may be deformed into a relatively thin and uniform shape, thereby facilitate an acquisition of a high-quality image.

To avoid the compression plate 141 from affecting an observation of a part of the patient by the physician, in one embodiment, the compression plate 141 may be a transparent plastic component, and a puncture hole may be arranged on the compression plate 141. The puncture hole may be used for the puncture of the puncture component 20. It should be understood that, in another embodiment, the compression plate 141 may also be non-transparent or include other transparent materials. When an inserting path of the needle does not pass through the compression plate 141, the puncture hole may also not be arranged on the compression plate 141.

A puncture component 20 may include a needle holder 21 and a puncture needle 22. The needle holder 21 may be used for an installation of the puncture needle 22. The puncture needle 22 may be used for the puncture of the breast 200 of the patient. In an actual operation process, the physician may determine a size and a position of the lesion 210 based on a captured image of the patient, and determine a need for coarse needle biopsy, fine needle biopsy, or other detection manners. When the coarse needle biopsy is needed, the puncture needle 22 may include a hollow coarse needle with a large inner diameter. A tissue specimen taken out by puncture may be sent to the Department of Pathology for fixation, dehydration, embedding, slicing, staining, and encapsulation to perform microscopic observation, thereby determining whether the tissue is pathologically diseased. The coarse needle biopsy may have a higher accuracy, and in comparison with surgical resection, a wound of the patient may be smaller and easier to recover. When the fine needle biopsy is needed, the puncture needle 22 may include a hollow fine needle with a small inner diameter, and cells taken out by puncture may be used for pathological diagnosis. A diagnosis speed may be faster, and the wound of the patient may be smaller, so that a recovery speed may be faster.

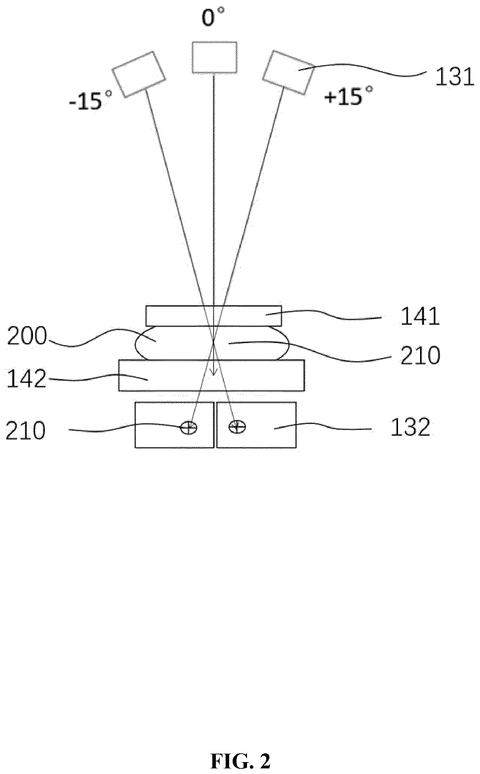

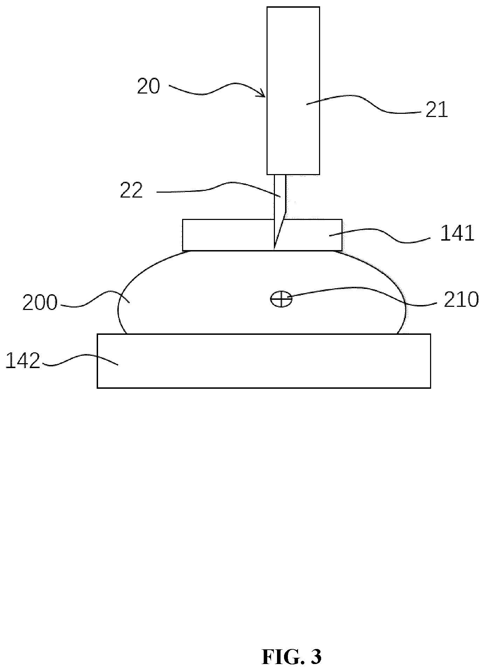

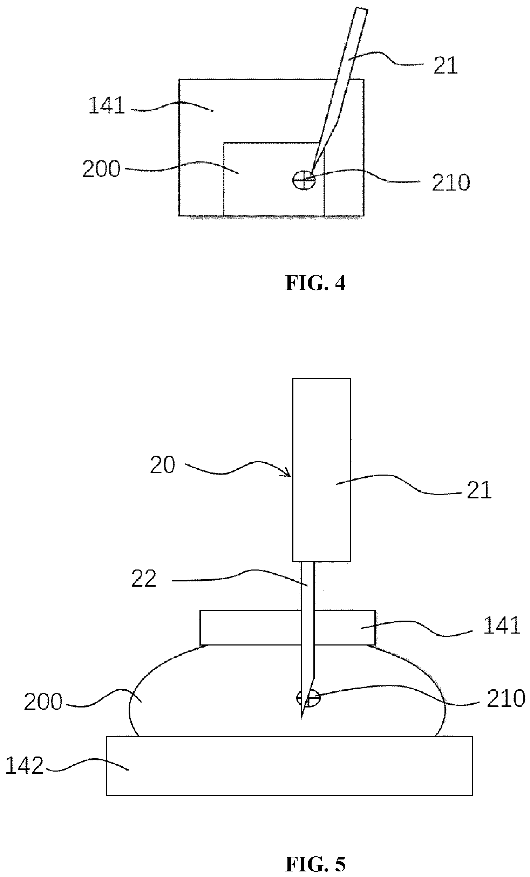

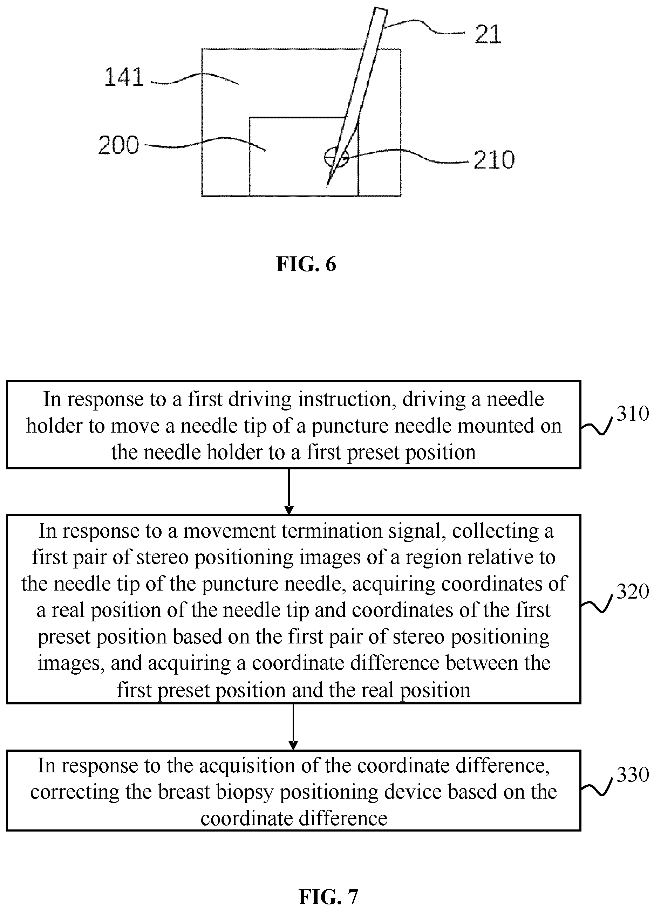

Referring to FIG. 1, and in combination with FIG. 2 through FIG. 6, FIG. 2 is a schematic diagram illustrating a principle of capturing an image of a scout frame and images of stereo pair frames by an imaging component; FIG. 3 is a schematic diagram illustrating capturing an image of a pre-fire frame by an imaging component; FIG. 4 is a schematic diagram illustrating an image of a pre-fire captured by an imaging component; FIG. 5 is a schematic diagram illustrating capturing an image of a post-fire frame by an imaging component; and FIG. 6 is a schematic diagram illustrating an image of a post-fire frame captured by an imaging component.

Usually, a process of the breast biopsy may be as follows: the breast 200 may be fixed using the compression component 14, so that the breast 200 may be deformed into the relatively thin and uniform shape; the photographing component may be set to zero degree (i.e., a connection line between the ray tube 131 and the detector 132 may be in a vertical direction). An image of a scout frame may be captured, whether the lesion 210 is clear may be determined by identifying whether a position is appropriate based on the image of the scout frame, and whether the lesion 210 is within a range of puncture may be determined. As shown in FIG. 2, the rotating support 12 may drive the photographing component to rotate, and an image of stereo pair frames may be captured at ±15 degrees (i.e., the connection line between the ray tube 131 and the detector 132 may deflect 15 degrees clockwise or counterclockwise relative to the vertical direction) respectively; stereo spatial position information of the lesion 210 in the breast 200 may be integrated and calculated based on image information of the image of the scout frame and the images of stereo pair frames, and the lesion 210 may be marked as a target point; an appropriate puncture needle 22 may be selected, and the puncture needle 22 may be mounted on the needle holder 21 and controlled to be inserted into the irradiated region 133, and the puncture needle 22 may be aligned with the target point. As shown in FIG. 3 and FIG. 4, before the puncture needle 22 is inserted into the breast 200 of the patient, an image of a pre-fire frame may be captured at ±15 degrees respectively to determine whether an inserting position of the puncture needle 22 is correct. In response to determining that the inserting position of the puncture needle 22 is deviated from a preset inserting position, the inserting position of the puncture needle 22 may be adjusted slightly, so that the puncture needle 22 may coincide with the preset inserting position; the puncture needle 22 may be inserted into the breast 200 of the patient, and the lesion 210 may be sampled through the puncture needle 22. As shown in FIG. 5 and FIG. 6, after the puncture needle 22 is emitted, an image of a post-fire frame may be captured at ±15 degrees respectively to determine whether the target point is located on the inserting path of the puncture needle 22, and determine whether the lesion 210 is located within a sample range of the puncture needle 22, thereby determining whether the lesion 210 is taken out; and the puncture needle 22 may be taken out from the breast 200, and the pathological examination may be performed on the tissue taken out by the puncture needle 22.

For a traditional breast device, the needle holder may be mounted on a gantry of the breast device, and arranged between a ray tube and a detector. To avoid the needle holder from being captured during imaging, when the image of the scout frame is captured, the needle holder may need to move to the farthest end to avoid the needle holder from entering the irradiated region. While the images of stereo pair frames, the image of the pre-fire frame, and the image of the post-fire frame are captured, the needle holder cannot be avoided being captured, so that part of the captured image may be blocked by the needle holder to affect diagnosis of the physician.

Accordingly, for the breast X-ray imaging apparatus 100 provided in the present disclosure, the needle holder 21 may move relative to the breast device 10, and the needle holder 21 may be arranged outside the irradiated region 133. Whether the image of the scout frame, or the images of stereo pair frames, the image of the pre-fire frame, and the image of the post-fire frame are captured, the needle holder 21 may be avoided from being captured by the photographing component 13, thereby avoiding the needle holder 21 from affecting the determination of a position of the lesion 210 by the physician.

It should be noted that, "the needle holder 21 may be arranged outside the irradiated region 133" in the present disclosure may refer to that the needle holder 21 may not be arranged within the irradiated region 133 formed by the ray tube 131 and the detector 132 when any image of the image of the scout frame, the images of stereo pair frames, the image of the pre-fire frame, and the image of the post-fire frame are captured. That is, the needle holder 21 may not be detected by the detector 132.

In one embodiment of the present disclosure, the breast X-ray imaging apparatus 100 may further include a mechanical arm 30. The needle holder 21 may be mounted on the mechanical arm 30, and the needle holder 21 may move relative to the breast device 10 under the driving of the mechanical arm 30. One end of the mechanical arm 30 may be fixed, and the other end of the mechanical arm 30 may be used for the installation of the needle holder 21. The mechanical arm 30 may drive the needle holder 21 to translate, rotate, or the like, to adjust a relative position of the needle holder 21. The mechanical arm 30 may be configured to drive the needle holder 21 to move, so that the movement of the needle holder 21 may be more flexible, thereby facilitating the needle holder 21 to be inserted at an optimal inserting angle during puncture to reduce the depth of the wound of the patient. In addition, the needle holder 21 may be mounted on the mechanical arm 30 to avoid repeated disassemble and assemble, which not only reduces an error caused by the installation of the needle holder 21, but also further reduces a calibration duration of the needle holder 21, thereby improving diagnosis efficiency.

It should be understood that, in another embodiment, the needle holder 21 may also be arranged on other mechanisms under the condition that the puncture operation may be achieved and the needle holder 21 may be avoided from entering the irradiated region 133.

To improve the flexibility of the mechanical arm 30, in one embodiment of the present disclosure, the mechanical arm 30 may include at least two arm joints 31, and the at least two arm joints 31 may be rotatably connected. An end of one arm joint 31 of the at least two arm joints 31 may be relatively fixed to the position of the gantry 11, and the needle holder 21 may be mounted on an end of another one arm joint 31 of the at least two arm joints 31, thereby improving the usage flexibility of the mechanical arm 30, which may be convenient for the physician to adjust the needle holder 21 and the puncture needle 22 to an appropriate inserting angle to improve the diagnosis efficiency.

It should be understood that, in another embodiment, if the flexibility of the mechanical arm 30 is not considered, the mechanical arm 30 may be merely configured with one arm joint 31 under the condition that the surgical operation may be achieved. In some embodiments, the mechanical arm 30 may also include three, four, or multiple arm joints 31, and the multiple arm joints 31 may be rotatably connected, fixedly connected, or retractably connected under the condition that a better movement performance may be achieved based on the mutual cooperation among the multiple arm joints 31.

In one embodiment of the present disclosure, at least one arm joint of the at least two arm joints 31 may be a retractable arm joint, thereby improving the usage flexibility of the mechanical arm 30.

In one embodiment of the present disclosure, the mechanical arm 30 may be arranged independently from the breast device 10, and a mounting position of the mechanical arm 30 may be relatively fixed to the position of the gantry 11. It should be noted that, "the mechanical arm 30 may be arranged independently from the breast device 10" may refer to that the mechanical arm 30 is not arranged on the breast device 10, but is arranged on other positions. In the present disclosure, the mechanical arm 30 may be fixed on a ward wall, ceiling, floor, or other positions that are independent from the breast device 10, thereby increasing a movement flexibility of the mechanical arm 30 relative to the breast device 10 and reducing a count of position calibrations of the mechanical arm 30. The mechanical arm 30 may not need to be calibrated before usage per time. That is, the mechanical arm 30 may be calibrated after a certain period of usage, and the time interval of the calibration period may be increased to 1 to 3 months.

It should be understood that, in another embodiment, the mechanical arm 30 may also be arranged on the breast device 10 under the condition that the mechanical arm 30 may drive the needle holder 21 to perform a surgical process and the needle holder 21 may be arranged outside the irradiated region 133.

In one embodiments of the present disclosure, the mechanical arm 30 may drive the puncture needle 22 to be inserted along a vertical direction, a horizontal direction, or a tilt direction relative to the compression plate 141. Since the breast 200 is a flexible tissue, the mutual cooperation between the compression plate 141 and the compression platform 142 may enable the breast 200 of the patient to be shaped and positioned, thereby avoiding a photographing error caused by a displacement of the breast 200. In addition, for a traditional breast device, a puncture needle may be merely inserted along a vertical direction or a horizontal direction. In the present disclosure, the needle holder 21 and the puncture 22 may move with the mechanical arm 30, thereby greatly increasing an inserting angle range and flexibility of the puncture needle 22. The puncture needle 22 may be inserted along the vertical direction, the horizontal direction, or the tilt direction, thereby reducing the depth of the wound of the patient.

A compression plate and a compression platform of a traditional breast device may usually rotate with a gantry. During an examination of a breast, after a rotation of a rotating support, the breast of the patient may need to be re-positioned, which extends examination time. In one embodiment of the present disclosure, the rotating support 12 may rotate relative to the compression plate 141 and the compression platform 142. During the examination, the breast 200 of the patient may not need to be re-positioned, and images from different angles may be captured based on the rotation of the rotating support 12.

It should be understood that, in another embodiment, if the examination time is not considered, the compression plate 141 and the compression platform 142 may rotate with the rotating support 12 under the condition that the surgical purpose is achieved.

In one embodiment of the present disclosure, the breast X-ray imaging apparatus 100 may further include a host, and the host may be communicatively connected with the mechanical arm 30 and configured to control a movement of the mechanical arm 30, which is convenient for an operation of the physician. There is no need to provide an additional device configured to control the movement of the mechanical arm 30, which reduce an occupied area of the breast X-ray imaging apparatus 100. It should be understood that, in another embodiment, the breast X-ray imaging apparatus 100 may be individually used under a determination and an operation of the physician.

In one embodiment of the present disclosure, the breast X-ray imaging apparatus 100 may further include a photographing unit 40. The photographing unit 40 may be communicatively connected with the host, and the needle holder 21 may be arranged outside the irradiated region 133 based on a visual navigation of the photographing unit 40. The photographing unit 40 may help the physician to observe a real time condition of the breast 10 during puncture to avoid uncomfortable of the patient. The photographing unit 40 may also cooperate with the host to guide the mechanical arm 30 to move and avoid obstacles through the visual navigation, and the mechanical arm 30 may drive the needle holder 21 and the puncture needle 22 to move to an optimal inserting position and perform the puncture operation. It should be understood that, in another embodiment, the photographing unit 40 may not be arranged, and the surgical operation may be achieved based on the determination and experience of the physician.

In some embodiments, after the optimal inserting position is determined, the host may calculate positions and inserting angles of the needle holder 21 and the puncture needle 22, and control the mechanical arm 30 to move, which may drive the needle holder 21 and the puncture needle 22 to move. The photographing unit 40 may collect image data in real time and transmit the image data to the host. The host may receive the image data and detect whether an obstacle exists in the image, determine position information of the obstacle, analyze and calculate the position information of the obstacle, re-arrange a moving path of the mechanical arm 30 to avoid the obstacle, and control the mechanical arm 30 to move along the re-arranged moving path to avoid the obstacle, so that the needle holder 21 and the puncture needle 22 may reach the optimal inserting position.

It should be noted that, the photographing unit 40 may include a 3D camera or a multi-camera device, thereby determining information about the obstacle.

Alternatively, the host may control the movement of the photographing unit 40, and a mounting position of the photographing unit 40 may be relatively fixed to the position of the gantry 11. The position of the photographing unit 40 may be relatively fixed, thereby increasing the time interval of the calibration period and making a process of the visual navigation more accurate.

For the breast X-ray imaging apparatus 100 provided in the present disclosure, the needle holder 21 may move relative to the breast 10. The needle holder 21 and the breast device 10 may be arranged outside the irradiated region 133 when the needle holder 21 and the breast device 10 are in a capture state before, during, and after puncture, that is, when the image of the scout frame, the images of stereo pair frames, the image of the pre-fire frame, and the image of the post-fire frame are captured. On the one hand, the breast X-ray imaging apparatus 100 provided in the present disclosure may optimize the image captured by the breast device 10 to avoid an appearance of the needle holder 21 in the the image captured before and after puncture, thereby achieving a better diagnosis of the position of the lesion 210 and preventing the lesion 210 from being blocked by the needle holder 21 during imaging. On the other hand, an operation efficiency of the physician may be improved, and the needle holder 21 may not need to be moved to the farthest end when capturing the image of the scout frame, thereby reducing a diagnosis period and avoiding a repeated capture caused by the physician forgetting to move the needle holder 21 to the farthest end before the image of the scout frame is captured.

With the continuous updating of imaging devices, digital breast tomosynthesis technology, also known as digital breast tomosynthesis (DBT), has emerged. A breast biopsy positioning device may be an important functional component of a DBT system. The breast biopsy positioning device may be an additional device independent from an overall structure of the DBT system morphologically, and the physician may activate the breast biopsy positioning device through manual installation. With an assistance of the breast biopsy positioning device, the physician may perform precise operations such as stereotactic positioning, puncture biopsy, or the like, on the patient.

The breast biopsy positioning device flexibly mounted on the DBT system may be mainly used for an accurate positioning of breast biopsy. In practical applications, the breast biopsy positioning device may be usually calibrated through manual calibration, and a relatively large deviation of a puncture position of the puncture needle for breast biopsy may result from a human error.

Accordingly, a method for correcting and verifying a breast biopsy positioning device may be provided in the present disclosure. The method may be applied to the breast X-ray imaging apparatus 100.

Referring to FIG. 7, FIG. 7 is a flowchart illustrating a process for correcting and verifying a breast biopsy positioning device according to an embodiment of the present disclosure. In the embodiment, the breast biopsy positioning device may be mounted on a breast X-ray imaging apparatus illustrated above. The breast biopsy positioning device may be connected to a needle holder. The method for correcting and verifying the breast biopsy positioning device may include the following operations.

In S310, in response to a first driving instruction, the needle holder may be driven to move a needle tip of a puncture needle mounted on the needle holder to a first preset position.

In the operation, the breast biopsy positioning device may include a driving device. In response to receiving the first driving instruction, the driving device may drive the needle holder to move. The first driving instruction may refer to an instruction generated by correcting the breast biopsy positioning device, which may be generated by triggering a touch signal or a key signal. The puncture needle may be clamped on the needle holder. The puncture needle may move with the movement of the needle holder, thereby moving the needle tip of the puncture needle to a preset position. The first preset position may be a fixed position selected based on a detection need of the breast. The needle holder may be driven to move automatically after the breast biopsy positioning device receives instruction information about the first preset position, so that the needle tip of the puncture needle may be moved to the first preset position.

In S320, in response to a movement termination signal, a first pair of stereo positioning images of a region relative to the needle tip of the puncture needle may be collected, coordinates of a real position of the needle tip and coordinates of the first preset position may be acquired based on the first pair of stereo positioning images, and a coordinate difference between the first preset position and the real position may also be acquired.

In the operation, after a driving process of the needle holder is terminated, the needle tip of the puncture needle may reach the first preset position. Corresponding state information may be reported to a system including the breast biopsy positioning device. The system may trigger a collection process of images to collect the first pair of stereo positioning images of the region where the needle tip of the puncture needle is located. The first pair of stereo positioning images may include at least two stereo positioning images which are captured at different scanning angles. The coordinates of the real position of the needle tip may be determined based on the first pair of stereo positioning images. Since the real position of the needle tip may deviate from the first preset position, the coordinate difference between the first preset position and the real position may be determined based on the first preset position and the real position of the needle tip identified and determined based on the first pair of stereo positioning images.

In S330, in response to the acquisition of the coordinate difference, the breast biopsy positioning device may be corrected based on the coordinate difference.

In the operation, after the coordinate difference is acquired, the breast biopsy positioning device may be corrected based on the coordinate difference, thereby reducing a deviation during a positioning process of the breast biopsy positioning device and improving a positioning accuracy of the breast biopsy positioning device in the system.

In the embodiment, during an accuracy correction of the breast biopsy positioning device, the needle holder of the breast biopsy positioning device may be driven to move based on a correction process and move the needle tip of the puncture needle to the first preset position. The accuracy correction of the breast biopsy positioning device may be achieved by measuring the real position of the needle tip and the first preset position, thereby reducing a human error introduced by personnel operation in determination and identification and improving an efficiency of the accuracy correction of the breast biopsy positioning device.

It should be noted that, the method for correcting and verifying the breast biopsy positioning device may be executed on a console or a post-processing workstation of the breast biopsy positioning device. The method may also be executed on a terminal or a computing device that can communicate with the breast biopsy positioning device. The method may also be executed on other breast examination devices except for the breast X-ray imaging apparatus 100, which is not limited herein. Changes and modifications may be performed based on actual application needs. For example, the method may be executed on the host of the breast X-ray imaging apparatus 100.

In some embodiments, after the driving process of the needle holder is terminated, the needle tip of the puncture needle may reach the first preset position. Corresponding state information may be reported to the system including the breast biopsy positioning device. The system may trigger a collecting process of images, e.g., an exposure workflow. Exposure data of different angles may be collected in the region where the needle tip of the puncture needle is located based on fixed exposure dose parameters of the system, and a pair of stereo positioning images may be acquired after the exposure data is processed based on an algorithm. Further, different angles may include two angles with a same absolute value, such as + 20 degrees, -20 degrees, or the like, and specific angles may be adjusted based on actual needs. Since a breast entity is not involved in the correction process, in practice operation, the compression plate for the breast may be removed, and then the correction process of the present disclosure may be performed.

In one embodiment, the acquiring coordinates of a real position of the needle tip and coordinates of the first preset position based on the first pair of stereo positioning images may include the following operations.

For each image of the first pair of stereo positioning images, coordinates of a first preset label may be identified, and the coordinates of the real position and the coordinates of the first preset position may be determined based on the coordinates of the first preset label. The each image of the first pair of stereo positioning images may include the first preset label of a same position. The real position of the needle tip of the puncture needle may include a target position. In another embodiment, the real position of the needle tip of the puncture needle may also include another position.

The correcting the breast biopsy positioning device based on the coordinate difference may include the following operations.

The breast biopsy positioning device may be corrected based on a coordinate difference corresponding to the each image of the first pair of stereo positioning images.

In the embodiment, for each image of the first pair of stereo positioning images, coordinates may be acquired respectively. When the first pair of stereo positioning images are captured, a certain position in the region where the needle tip of the puncture needle is located may be provided with the first preset label. The first preset label may be displayed in the each image of the first pair of stereo positioning images. The first preset label may serve as a correlation reference point, coordinates of a position of the correlation reference point may be correlated with the coordinates of the first preset position to establish a position association between the real position and the first preset position. The breast biopsy positioning device may be corrected based on the coordinate difference between the real position and the first preset position.

In some embodiments, the first preset label may include but not limited to a label on an object stage of the breast biopsy positioning device, or a label of another position in the irradiated region. Before the coordinates of the first preset label are identified, a scale of a current image may be adjusted to fully display the current image. In addition, coordinate differences of images of the first pair of stereo positioning images may be averaged or weighted-averaged to obtain a correction compensation value of the first pair of stereo positioning images, and the breast biopsy positioning device may be corrected based on the correction compensation value.

In one embodiment, when there is a plurality of first preset positions, the method for correcting and verifying the breast biopsy positioning device may also include the following operations.

The needle tip of the puncture needle may be moved to a next first preset position after the coordinate difference at a current first preset position is acquired.

A correction compensation value of the breast biopsy positioning device may be acquired based on coordinate differences obtained at the plurality of first preset positions, and the breast biopsy positioning device may be corrected based on the correction compensation value.

In the embodiment, coordinate differences may be acquired at the plurality of first preset positions. During an actual operation, a process may be performed at different first preset positions in sequence. The coordinate differences of the plurality of first preset positions may be configured to comprehensively determine the correction compensation value of the breast biopsy positioning device, thereby improving an accuracy of the accuracy correction of the breast biopsy positioning device.

Further, a count of the first preset positions may include two or more than three. After a coordinate difference of each first preset position is acquired, a plurality of coordinate differences may be averaged or weighted-averaged to determine the correction compensation value.

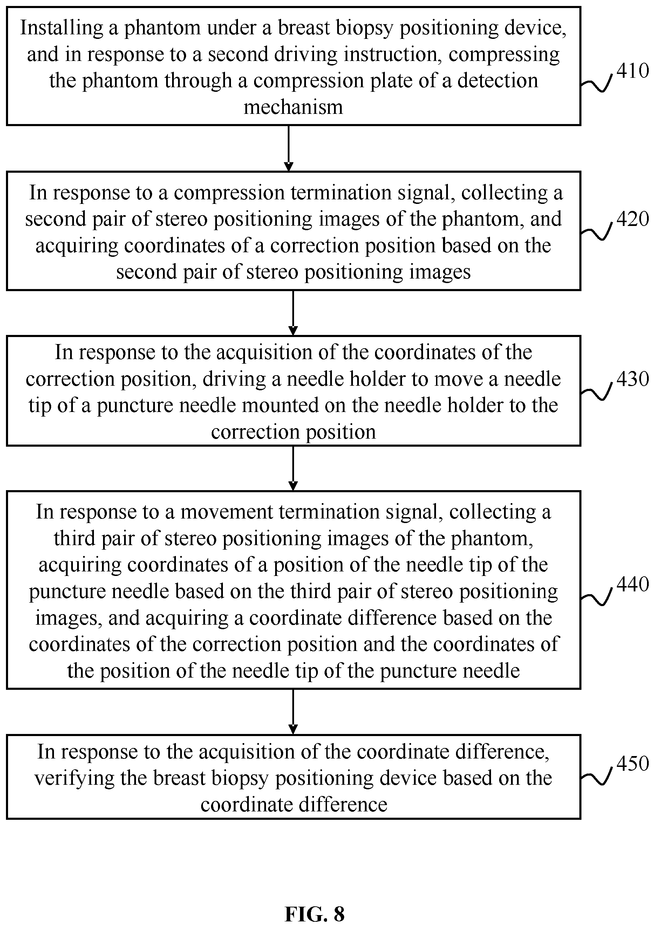

Referring to FIG. 8, FIG. 8 is a flowchart illustrating a process for correcting and verifying a breast biopsy positioning device according to another embodiment of the present disclosure. In the embodiment, the breast biopsy positioning device may be mounted on a breast X-ray imaging apparatus illustrated above. The breast biopsy positioning device may be connected with a needle holder. A method for correcting and verifying a breast biopsy positioning device may include the following operations.

In 410, a phantom may be installed under the breast biopsy positioning device, and in response to a second driving instruction, the phantom may be compressed through a compression plate of a detection mechanism.

In the operation, a breast phantom may be selected to be installed. In response to that the second driving instruction is received, the compression plate 141 of the detection mechanism may be configured to compress the breast phantom to simulate a practical application scenario of the breast biopsy positioning device. An installation of the phantom and a compression of the compression plate 141 may be performed by a driving accessory of the breast biopsy positioning device. The second driving instruction may be an instruction generated by identifying the breast biopsy positioning device. The generation of the second driving instruction may be triggered by a touch signal or a key signal.

In 420, in response to a compression termination signal, a second pair of stereo positioning images of the phantom may be collected, and coordinates of a correction position may be acquired based on the second pair of stereo positioning images.

In the operation, after a compression simulation is terminated, corresponding state information may be reported to a system including the breast biopsy positioning device. The system may trigger a collection process of images to collect the second pair of stereo positioning images of a region where the phantom is located. The second pair of stereo positioning images may include at least two stereo positioning images which are captured at different scanning angles. The coordinates of the correction position of a tissue that needs to be taken out, that is, a position that a needle tip of the puncture needle actually needs to reach, may be determined based on the second pair of stereo positioning images.

In 430, in response to the acquisition of the coordinates of the correction position, the needle holder may be driven to move the needle tip of the puncture needle mounted on the needle holder to the correction position.

In the operation, the breast biopsy positioning device may include a driving device. The driving device may drive the needle holder to move after the coordinates of the correction position are determined. A puncture needle may be clamped on the needle holder. The puncture needle may move with the movement of the needle holder, thereby the needle tip of the puncture needle may be moved to the correction position.

In 440, in response to a movement termination signal, a third pair of stereo positioning images of the phantom may be collected, coordinates of a position of the needle tip of the puncture needle may be acquired based on the third pair of stereo positioning images, and a coordinate difference may be acquired based on the coordinates of the correction position and the coordinates of the position of the needle tip of the puncture needle.

In the operation, after a driving process of the needle holder is terminated, the needle tip of the puncture needle may reach the correction position. Corresponding state information may be reported to the system including the breast biopsy positioning device. The system may trigger a collection process of images to collect the third pair of stereo positioning images of a region where the needle tip of the puncture needle is located. The third pair of stereo positioning images may include at least two stereo positioning images which are captured at different scanning angles. The coordinates of the correction position and the coordinates of the position of the needle tip may be determined based on the third pair of stereo positioning images. Since changes of a spatial position of the phantom may be caused by the puncture process, the position of the needle tip may deviate from the correction position, and the coordinate difference between the position of the needle tip and the correction position may be determined by identifying the third pair of stereo positioning images.

In 450, in response to the acquisition of the coordinate difference, the breast biopsy positioning device may be verified based on the coordinate difference.

In the operation, after the coordinate difference is acquired, the breast biopsy positioning device may be verified based on the coordinate difference to verify whether the breast biopsy positioning device satisfies a corresponding desired accuracy.

In the embodiment, during an accuracy verification of the breast biopsy positioning device, the needle holder of the breast biopsy positioning device may be driven to move based on a verification process to determine the correction position for the verification. The needle tip of the puncture needle may be moved to the correction position, and a pair of stereo positioning images may be collected. The accuracy verification of the breast biopsy positioning device may be achieved by measuring the position of the needle tip and the correction position, thereby reducing a human error introduced by personnel operation in determination and identification and improving an efficiency of the accuracy correction of the breast biopsy positioning device.

It should be noted that, the method for correcting and verifying the breast biopsy positioning device may be executed on a console or a post-processing workstation of the breast biopsy positioning device. The method may also be executed on a terminal or a computing device that may be in communication with the breast biopsy positioning device, which is not limited herein. Changes and modifications may be performed based on actual application requirements. For example, the method may be executed on the host of the breast X-ray imaging apparatus 100.

In some embodiments, the system may trigger a collection process of images, which is an exposure workflow. Exposure data of different angles may be collected in the region where the phantom is located based on fixed exposure dose parameters of the system, and a pair of stereo positioning images may be acquired after the exposure data is processed based on an algorithm. Further, different angles may be two angles with a same absolute value, such as + 20 degrees, -20 degrees, or the like, and a specific angle may be adjusted based on actual needs.

In one embodiment, the acquiring coordinates of a correction position based on the second pair of stereo positioning images may include the following operations.

For each image of the second pair of stereo positioning images, coordinates of a second preset label may be identified, and the coordinates of the correction position may be determined based on the coordinates of the second preset label. The each image of the second pair of stereo positioning images may include the second preset label of a same position.

The acquiring coordinates of a position of the needle tip of the puncture needle based on the third pair of stereo positioning images may include the following operations.

For each image of the third pair of stereo positioning images, the coordinates of the second preset label may be identified, and the coordinates of the position of the needle tip of the puncture needle may be determined based on the coordinates of the second preset label. The each image of the third pair of stereo positioning images may include the second preset label of a same position.

In the embodiment, for each image of the second pair of stereo positioning images, coordinates may be acquired respectively. When the second pair of stereo positioning images are captured, a certain real position in the region where the phantom is located may be provided with the second preset label. The second preset label may be displayed in the each image of the second pair of stereo positioning images. The second preset label may serve as a reference point configured to determine the coordinates of the correction position. For the third pair of stereo positioning images, coordinates of the position of the needle tip of the puncture needle may be determined in a similar way. The breast biopsy positioning device may be verified based on the coordinate difference between the correction position and the position of the needle tip of the puncture needle.

In some embodiments, the second preset label may include but not limited to a label on an object stage of the breast biopsy positioning device, or a label of another position in the irradiated region. Before the coordinates of the second preset label are identified, a scale of a current image may be adjusted to fully display the current image. In addition, coordinates of correction positions in images of the second pair of stereo positioning images may be averaged or weighted-averaged to obtain the coordinates of the correction position corresponding to the second pair of stereo positioning images, and coordinates of positions of the needle tip in images of the third pair of stereo positioning images may be averaged or weighted-averaged to obtain the coordinates of the position of the needle tip corresponding to the third pair of stereo positioning images. An accuracy of correcting the breast biopsy positioning device based on the correction compensation value may be determined based on the coordinates of the correction position and the coordinates of the position of the needle tip.

In one embodiment, there may be a plurality of correction positions. The method for correcting and verifying a breast biopsy positioning device may include the following operations.

The needle tip of the puncture needle may be moved to a next correction position after the breast biopsy positioning device at a current correction position is verified.

A target verification result of the breast biopsy positioning device may be acquired based on verification results obtained at the plurality of correction positions.

In the one embodiment, the verification process may be performed on the plurality of correction positions. During an actual operation, a process may be performed at different correction positions in sequence. The verification results of the plurality of correction positions may be configured to comprehensively verify an overall accuracy level of the breast biopsy positioning device.

Further, a count of the correction positions may include two and more than three.

It should be noted that, the method for correcting and verifying a breast biopsy positioning device may include two processes of correction and verification. After the correction process is terminated, the verification process may be performed. A mutual cooperation between the two processes may be achieved, which is not contradictory.

According to the method for correcting and verifying a breast biopsy positioning device, the embodiments of the present disclosure further provide a system for correcting and verifying a breast biopsy positioning device. Detailed illustrations of the system for correcting and verifying a breast biopsy positioning device may refer to the following descriptions about the embodiments.

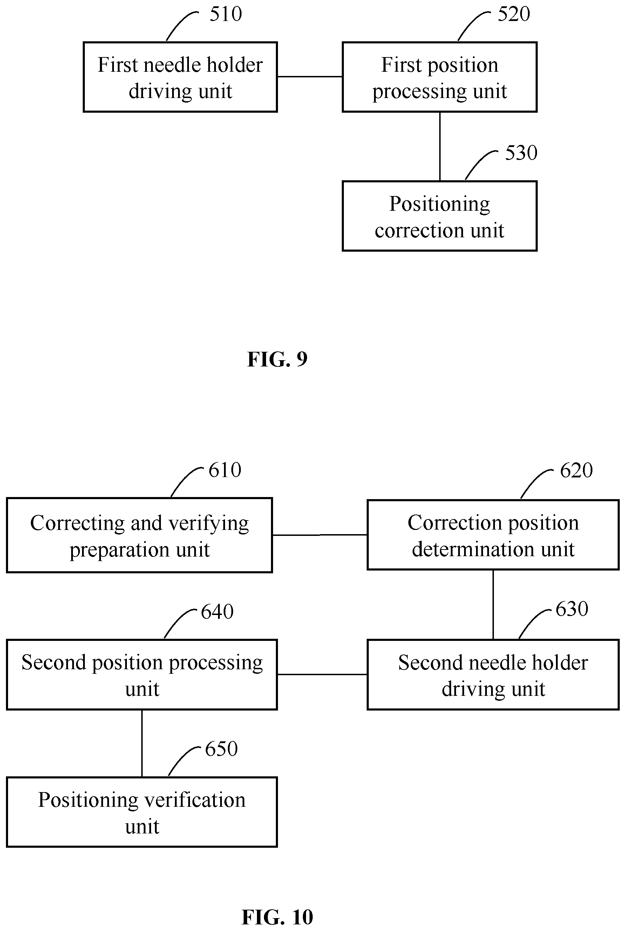

Referring to FIG. 9, FIG. 9 is a schematic diagram illustrating a structure of a system for correcting and verifying a breast biopsy positioning device according to an embodiment of the present disclosure. In the embodiment, the breast biopsy positioning device may be mounted on a breast X-ray imaging apparatus illustrated above. The breast biopsy positioning device may be connected with a needle holder. The system for correcting and verifying a breast biopsy positioning device may include:
a first needle holder driving unit 510, in response to a first driving instruction, configured to drive the needle holder to move a needle tip of a puncture needle mounted on the needle holder to a first preset position;
a first position processing unit 520, in response to a movement termination signal, configured to collect a first pair of stereo positioning images of a region relative to the needle tip of the puncture needle, acquire, based on the first pair of stereo positioning images, coordinates of a real position of the needle tip and coordinates of the first preset position, and acquire a coordinate difference between the first preset position and the real position;
a positioning correction unit 530, in response to the acquisition of the coordinate difference, configured to correct the breast biopsy positioning device based on the coordinate difference.

In one embodiment, for each image of the first pair of stereo positioning images, the first position processing unit 520 may be configured to identify coordinates of a first preset label, and determine the coordinates of the real position and the coordinates of the first preset position based on the coordinates of the first preset label. The each image of the first pair of stereo positioning images may include the first preset label of a same position.

The positioning correction unit 530 may be configured to correct the breast biopsy positioning device based on a coordinate difference corresponding to the each image of the first pair of stereo positioning images.

In one embodiment, there may be a plurality of first preset positions. The first needle holder driving unit 510 may move the needle tip of the puncture needle to a next first preset position after the positioning correction unit 530 acquires the coordinate difference at a current first preset position.

The positioning correction unit 530 may also be configured to acquire a correction compensation value of the breast biopsy positioning device based on coordinate differences obtained at the plurality of first preset positions, and correct the breast biopsy positioning device based on the correction compensation value.

Referring to FIG. 10, FIG. 10 is a schematic diagram illustrating a structure of a system for correcting and verifying a breast biopsy positioning device according to another embodiment of the present disclosure. In the embodiment, the breast biopsy positioning device may be mounted on a breast X-ray imaging apparatus illustrated above, and the breast biopsy positioning device may be connected with a needle holder. The system for correcting and verifying a breast biopsy positioning device may include:
a correcting and verifying preparation unit 610 configured to install a phantom under the breast biopsy positioning device, and in response to a second driving instruction, compress the phantom through a compression plate;
a correction position determination unit 620, in response to a compression termination signal, configured to collect a second pair of stereo positioning images of the, and acquire coordinates of a correction position based on the second pair of stereo positioning images;
a second needle holder driving unit 630, in response to the acquisition of the coordinates of the correction position, configured to drive the needle holder to move the needle tip of the puncture needle mounted on the needle holder to the correction position;
a second position processing unit 640, in response to a movement termination signal, configured to collect a third pair of stereo positioning images of the phantom, acquire coordinates of a position of the needle tip of the puncture needle based on the third pair of stereo positioning images, and acquire a coordinate difference based on the coordinates of the correction position and the coordinates of the position of the needle tip of the puncture needle;
a positioning verification unit 650, in response to the acquisition of the coordinate difference, configured to verify the breast biopsy positioning device based on the coordinate difference.

In one embodiment, for each image of the second pair of stereo positioning images, the correction position determination unit 620 may also be configured to identify coordinates of a second preset label, and determine the coordinates of the correction position based on the coordinates of the second preset label. The each image of the second pair of stereo positioning images may include the second preset label of a same position.

For each image of the third pair of stereo positioning images, the second position processing unit 640 may also be configured to identify the coordinates of the second preset label, and determine the coordinates of the position of the needle tip of the puncture needle based on the coordinates of the second preset label. The each image of the third pair of stereo positioning images may include the second preset label of a same position.

In one embodiment, there may be a plurality of correction positions. The second needle holder driving unit 630 may move the needle tip of the puncture needle to a next correction position after the positioning verification unit 650 verifies the breast biopsy positioning device at a current correction position.

The positioning verification unit 650 may also be configured to acquire a target verification result of the breast biopsy positioning device based on verification results obtained at the plurality of correction positions.

The system for correcting and verifying a breast biopsy positioning device provided in the embodiments of the present disclosure may correspond to the method for correcting and verifying a breast biopsy positioning device illustrated above. Technical features and beneficial effects of the method for correcting and verifying a breast biopsy positioning device illustrated above may also be applicable to the embodiments of the system for correcting and verifying a breast biopsy positioning device.

The present disclosure also provides a non-transitory computer readable medium comprising a set of instructions. When executed by at least one processor, the set of instructions may direct the at least one processor to effectuate the operations in the method for correcting and verifying a breast biopsy positioning device.

For the non-transitory computer readable medium mentioned above, during an accuracy correction and verification of the breast biopsy positioning device, the needle holder of the breast biopsy positioning device may be driven to move based on different workflows of the correction process and the verification process to move the needle tip of the puncture needle to a preset position, and a pair of stereo positioning images may be collected and identified, which is achieved based on the set of instructions stored on the non-transitory computer readable medium. The accuracy correction and verification of the breast biopsy positioning device may be achieved by measuring the position of the needle tip and the preset position, thereby reducing a human error introduced by personnel operation in determination and identification and improving an efficiency of the accuracy correction and verification of the breast biopsy positioning device.

The present disclosure may also provide an apparatus for correcting and verifying a breast biopsy positioning device. The apparatus may include at least one processor and at least one storage device. The at least one storage device may include a set of instructions. When executed by the at least one processor, the set of instructions may direct the at least one processor to effectuate the operations of the method for correcting and verifying a breast biopsy positioning device.

For the apparatus for correcting and verifying a breast biopsy positioning device mentioned above, during an accuracy correction and verification of the breast biopsy positioning device, the needle holder of the breast biopsy positioning device may be driven to move based on different workflows of the correction process and the verification process to move the needle tip of the puncture needle to a preset position, and a pair of stereo positioning images may be collected and identified, which is achieved based on the set of instructions stored on the non-transitory computer readable medium. The accuracy correction and verification of the breast biopsy positioning device may be achieved by measuring the position of the needle tip and the preset position, thereby reducing a human error introduced by personnel operation in determination and identification and improving an efficiency of the accuracy correction and verification of the breast biopsy positioning device.

Those skilled in the art may understand that an overall or part of workflows of a method for correcting and verifying a breast biopsy positioning device may be achieved by instructing related hardware through computer programs. The computer programs may be stored in a non-volatile computer readable storage medium. For example, in the embodiments, the computer programs may be stored in the storage medium of a computer system and executed by at least one processor in the computer system to realize the workflows including the method for correcting and verifying a breast biopsy positioning device. The storage medium may be disk, disc, read-only memory (ROM), random access memory (RAM), or the like.

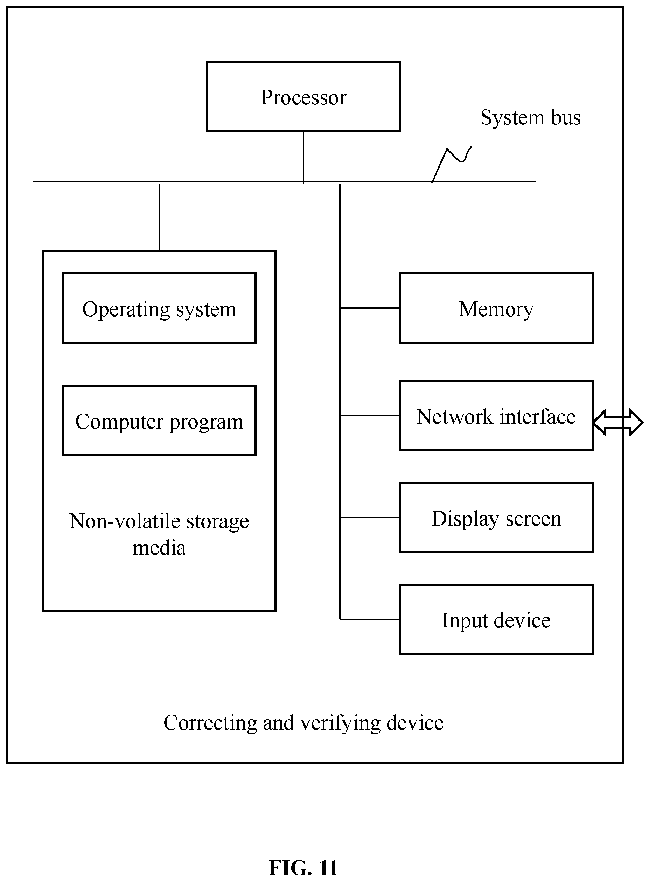

A correcting and verifying device of the apparatus for correcting and verifying a breast biopsy positioning device may be a terminal. An internal structure of the correcting and verifying device may be shown as FIG. 11. The correcting and verifying device may include a processor, a storage device, a network interface, a display screen, and an input device that are connected through a system bus. The processor of the correcting and verifying device may be configured to provide a capability of calculation and controllability. The storage device of the correcting and verifying device may include non-volatile storage media and memory. The non-volatile storage media may store operating systems and computer programs. The memory may provide an environment for the operation of the operating systems and the computer programs stored in the non-volatile storage media. The network interface of the correcting and verifying device may be configured to communicate with external terminals through network connection. The computer programs may be executed by the processor to achieve the method for correcting and verifying a breast biopsy positioning device. The display screen of the correcting and verifying device may include a liquid crystal display screen or an electronic ink display screen. The input device of the correcting and verifying device may include a touch layer covered on the display screen, or a key, a trackball, or a touchpad set on a shell of the correcting and verifying device. The input device of the correcting and verifying device may also include an external keyboard, an external touchpad, an external mouse, or the like.

Those skilled in the art may also understand that the structure shown in FIG. 11 may be merely a block diagram illustrating part of the structure that is related to the embodiments of the present disclosure, which is not intended to limit the correcting and verifying device applied to the embodiments of the present disclosure. A specific correcting and verifying device may include more or less components compared with the components shown in FIG. 11. A specific correcting and verifying device may also combine certain components. A specific correcting and verifying device may also include different arrangements of the components.

The particular features, structures, or characteristics may be combined as suitable in one or more embodiments of the present disclosure. For a concise description, possible combinations of the particular features in the embodiments mentioned above may not be described. However, it should be considered that the possible combinations of the particular features may be within the scope of the present disclosure under the condition that there is no contradiction in the possible combinations of the particular features.

The embodiments mentioned above may merely describe several implementation manners of the present disclosure. The descriptions may be more specific and include more details. However, it should be understood that the specific and detailed descriptions may not limit the scope of the present disclosure.

It should be noted that, various alterations, improvements, and modifications may occur and are intended to those skilled in the art, though not expressly stated herein. These alterations, improvements, and modifications are intended to be suggested by this disclosure, and are within the scope of the exemplary embodiments of this disclosure. Therefore, the scope of the present disclosure may be subject to the attached claims.

## Claims

1. A breast X-ray imaging apparatus (100), comprising:
a detection mechanism and a needle holder (21), wherein
the detection mechanism includes a ray tube (131) and a detector (132) that is arranged opposite to the ray tube (131), an irradiated region (133) being arranged between the ray tube (131) and the detector (132); and
the needle holder (21) is configured to move relative to the detection mechanism, and the needle holder (21) is arranged outside the irradiated region (133); and
a mechanical arm (30), the needle holder (21) being mounted on the mechanical arm (30), and the mechanical arm (30) being configured to drive the needle holder (21) to move relative to the detection mechanism, wherein
the detection mechanism further includes a gantry (11), the ray tube (131) and the detector (132) being mounted on the gantry (11); **characterized in that**
the mechanical arm (30) is arranged independently from the detection mechanism, a mounting position of the mechanical arm (30) being relatively fixed to a position of the gantry (11); wherein
the breast X-ray imaging apparatus (100) further comprises a host and a photographing unit (40), wherein the host is communicatively connected with the mechanical arm (30) and configured to control a movement of the mechanical arm (30), and the photographing unit (40) is communicatively connected with the host, and the needle holder (21) is arranged outside the irradiated region (133) based on a visual navigation of the photographing unit (40).

2. The breast X-ray imaging apparatus (100) of claim 1, wherein
the detection mechanism further includes a compression plate (141) and a compression platform (142), wherein the compression plate (141) and the compression platform (142) are mounted on the gantry (11), the compression plate (141) and the compression platform (142) are arranged between the ray tube (131) and the detector (132), and the compression plate (141) is arranged opposite to the compression platform (142), and
the breast X-ray imaging apparatus (100) further includes a puncture needle (22), wherein the puncture needle (22) is mounted on the needle holder (21), and the mechanical arm (30) is configured to drive the puncture needle (22) to be inserted along a tilt direction relative to the compression plate (141).

3. The breast X-ray imaging apparatus (100) of claim 2, wherein the detection mechanism further includes a rotating support (12), wherein
the rotating support (12) is rotatably mounted on the gantry (11), the ray tube (131) and the detector (132) are mounted on two ends of an opening of the rotating support (12), and the rotating support (12) is configured to rotate relative to the compression plate (141) and the compression platform (142).

4. The breast X-ray imaging apparatus (100) of any one of claims 1-3,
wherein the mechanical arm (30) includes at least two arm joints (31), the at least two arm joints (31) being rotatably connected, wherein
an end of one arm joint (31) of the at least two arm joints (31) is relatively fixed to the position of the gantry (11), and the needle holder (21) is mounted on an end of another one arm joint (31) of the at least two arm joints (31).

5. The breast X-ray imaging apparatus (100) of claim 4, wherein at least one of the at least two arm joints (31) is a retractable arm joint.

6. The breast X-ray imaging apparatus (100) of claim 1, wherein the host is further configured to control a movement of the photographing unit (40), and a mounting position of the photographing unit (40) is relatively fixed to the position of the gantry (11).

7. A method for correcting and verifying a breast biopsy positioning device, the breast biopsy positioning device being mounted on a breast X-ray imaging apparatus (100), wherein the breast X-ray imaging apparatus (100) includes a detection mechanism and a needle holder (21), the detection mechanism including a ray tube (131) and a detector (132) that is arranged opposite to the ray tube (131), an irradiated region (133) being arranged between the ray tube (131) and the detector (132), the needle holder (21) being configured to move relative to the detection mechanism, and the needle holder (21) being arranged outside the irradiated region (133); and wherein the breast X-ray imaging apparatus (100) further includes a mechanical arm (30), the needle holder (21) being mounted on the mechanical arm (30), and the mechanical arm (30) being configured to drive the needle holder (21) to move relative to the detection mechanism; wherein the detection mechanism further includes a gantry (11), the ray tube (131) and the detector (132) being mounted on the gantry (11), **characterized in that** the mechanical arm (30) is arranged independently from the detection mechanism, a mounting position of the mechanical arm (30) being relatively fixed to a position of the gantry (11), and the breast biopsy positioning device is connected with the needle holder (21), wherein
the breast X-ray imaging apparatus (100) further includes a host and a photographing unit (40), wherein the host is communicatively connected with the mechanical arm (30) and configured to control a movement of the mechanical arm (30), and the photographing unit (40) is communicatively connected with the host, and the needle holder (21) is arranged outside the irradiated region (133) based on a visual navigation of the photographing unit (40),
and the method comprises:
in response to a first driving instruction, driving (310) the needle holder (21) to move a needle tip of a puncture needle (22) mounted on the needle holder (21) to a first preset position;
in response to a movement termination signal, collecting (320) a first pair of stereo positioning images of a region relative to the needle tip of the puncture needle (22), acquiring (320), based on the first pair of stereo positioning images, coordinates of a real position of the needle tip and coordinates of the first preset position, and acquiring (320) a coordinate difference between the first preset position and the real position; and
in response to the acquisition of the coordinate difference, correcting (330) the breast biopsy positioning device based on the coordinate difference.

8. The method of claim 7, wherein
the acquiring coordinates of a real position of the needle tip and coordinates of the first preset position based on the first pair of stereo positioning images includes:
for each image of the first pair of stereo positioning images, identifying coordinates of a first preset label, and determining the coordinates of the real position and the coordinates of the first preset position based on the coordinates of the first preset label, wherein the each image of the first pair of stereo positioning images includes the first preset label of a same position; and
the correcting the breast biopsy positioning device based on the coordinate difference includes:
correcting the breast biopsy positioning device based on a coordinate difference corresponding to the each image of the first pair of stereo positioning images.

9. The method of claim 7 or 8, wherein there are a plurality of first preset positions, and the method further includes:
moving the needle tip of the puncture needle (22) to a next first preset position after acquiring the coordinate difference at a current first preset position; and
acquiring a correction compensation value of the breast biopsy positioning device based on coordinate differences obtained at the plurality of first preset positions, and correcting the breast biopsy positioning device based on the correction compensation value.

## Patentansprüche

1. Brust-Röntgenbildgebungseinrichtung (100), umfassend:
einen Detektionsmechanismus und einen Nadelhalter (21), wobei
der Detektionsmechanismus eine Strahlröhre (131) und einen Detektor (132) beinhaltet, der der Strahlröhre (131) gegenüberliegend angeordnet ist, wobei ein bestrahlter Bereich (133) zwischen der Strahlröhre (131) und dem Detektor (132) angeordnet ist; und
der Nadelhalter (21) so konfiguriert ist, dass er sich relativ zum Detektionsmechanismus bewegt, und der Nadelhalter (21) außerhalb des bestrahlten Bereichs (133) angeordnet ist; und
einen mechanischen Arm (30), wobei der Nadelhalter (21) an dem mechanischen Arm (30) angebracht ist und der mechanische Arm (30) so konfiguriert ist, dass er den Nadelhalter (21) antreibt, um ihn relativ zum Detektionsmechanismus zu bewegen, wobei
der Detektionsmechanismus weiter eine Gantry (11) beinhaltet, wobei die Strahlröhre (131) und der Detektor (132) an der Gantry (11) angebracht sind; **dadurch gekennzeichnet, dass**
der mechanische Arm (30) unabhängig vom Detektionsmechanismus angeordnet ist, wobei eine Anbringungsposition des mechanischen Arms (30) relativ zu einer Position der Gantry (11) fest ist; wobei
die Brust-Röntgenbildgebungseinrichtung (100) weiter einen Host und eine Fotografiereinheit (40) umfasst, wobei der Host kommunikativ mit dem mechanischen Arm (30) verbunden und so konfiguriert ist, dass er eine Bewegung des mechanischen Arms (30) steuert, und die Fotografiereinheit (40) kommunikativ mit dem Host verbunden ist, und der Nadelhalter (21) auf Basis einer visuellen Navigation der Fotografiereinheit (40) außerhalb des bestrahlten Bereichs (133) angeordnet ist.

2. Brust-Röntgenbildgebungseinrichtung (100) nach Anspruch 1, wobei
der Detektionsmechanismus weiter eine Kompressionsplatte (141) und eine Kompressionsplattform (142) beinhaltet, wobei die Kompressionsplatte (141) und die Kompressionsplattform (142) an der Gantry (11) angebracht sind, die Kompressionsplatte (141) und die Kompressionsplattform (142) zwischen der Strahlröhre (131) und dem Detektor (132) angeordnet sind und die Kompressionsplatte (141) der Kompressionsplattform (142) gegenüberliegend angeordnet ist, und
die Brust-Röntgenbildgebungseinrichtung (100) weiter eine Punktionsnadel (22) beinhaltet, wobei die Punktionsnadel (22) am Nadelhalter (21) angebracht ist und der mechanische Arm (30) so konfiguriert ist, dass er die Punktionsnadel (22) antreibt, um sie entlang einer Neigungsrichtung relativ zur Kompressionsplatte (141) einzuführen.

3. Brust-Röntgenbildgebungseinrichtung (100) nach Anspruch 2, wobei der Detektionsmechanismus weiter einen Drehträger (12) beinhaltet, wobei
der Drehträger (12) drehbar an der Gantry (11) angebracht ist, die Strahlröhre (131) und der Detektor (132) an zwei Enden einer Öffnung des Drehträgers (12) angebracht sind und der Drehträger (12) so konfiguriert ist, dass er sich relativ zur Kompressionsplatte (141) und der Kompressionsplattform (142) dreht.

4. Brust-Röntgenbildgebungseinrichtung (100) nach einem der Ansprüche 1-3, wobei der mechanische Arm (30) mindestens zwei Armgelenke (31) beinhaltet, wobei die mindestens zwei Armgelenke (31) drehbar verbunden sind, wobei
ein Ende eines Armgelenks (31) der mindestens zwei Armgelenke (31) relativ zur Position der Gantry (11) fest ist, und der Nadelhalter (21) an einem Ende eines anderen Armgelenks (31) der mindestens zwei Armgelenke (31) angebracht ist.

5. Brust-Röntgenbildgebungseinrichtung (100) nach Anspruch 4, wobei mindestens eines der mindestens zwei Armgelenke (31) ein einfahrbares Armgelenk ist.

6. Brust-Röntgenbildgebungseinrichtung (100) nach Anspruch 1, wobei der Host weiter so konfiguriert ist, dass er eine Bewegung der Fotografiereinheit (40) steuert, und eine Anbringungsposition der Fotografiereinheit (40) relativ zur Position der Gantry (11) fest ist.

7. Verfahren zum Korrigieren und Verifizieren einer Brustbiopsie-Positionierungsvorrichtung, wobei die Brustbiopsie-Positionierungsvorrichtung an einer Brust-Röntgenbildgebungseinrichtung (100) angebracht ist, wobei die Brust-Röntgenbildgebungseinrichtung (100) einen Detektionsmechanismus und einen Nadelhalter (21) beinhaltet, wobei der Detektionsmechanismus eine Strahlröhre (131) und einen Detektor (132) beinhaltet, der der Strahlröhre (131) gegenüberliegend angeordnet ist, wobei ein bestrahlter Bereich (133) zwischen der Strahlröhre (131) und dem Detektor (132) angeordnet ist, wobei der Nadelhalter (21) so konfiguriert ist, dass er sich relativ zum Detektionsmechanismus bewegt, und wobei der Nadelhalter (21) außerhalb des bestrahlten Bereichs (133) angeordnet ist; und wobei die Brust-Röntgenbildgebungseinrichtung (100) weiter einen mechanischen Arm (30) beinhaltet, wobei der Nadelhalter (21) an dem mechanischen Arm (30) angebracht ist und der mechanische Arm (30) so konfiguriert ist, dass er den Nadelhalter (21) antreibt, um ihn relativ zum Detektionsmechanismus zu bewegen; wobei der Detektionsmechanismus weiter eine Gantry (11) beinhaltet, wobei die Strahlröhre (131) und der Detektor (132) an der Gantry (11) angebracht sind, **dadurch gekennzeichnet, dass** der mechanische Arm (30) unabhängig vom Detektionsmechanismus angeordnet ist, wobei eine Anbringungsposition des mechanischen Arms (30) relativ zu einer Position der Gantry (11) fest ist, und die Brustbiopsie-Positionierungsvorrichtung mit dem Nadelhalter (21) verbunden ist, wobei
die Brust-Röntgenbildgebungseinrichtung (100) weiter einen Host und eine Fotografiereinheit (40) beinhaltet, wobei der Host kommunikativ mit dem mechanischen Arm (30) verbunden und so konfiguriert ist, dass er eine Bewegung des mechanischen Arms (30) steuert, und die Fotografiereinheit (40) kommunikativ mit dem Host verbunden ist, und der Nadelhalter (21) auf Basis einer visuellen Navigation der Fotografiereinheit (40) außerhalb des bestrahlten Bereichs (133) angeordnet ist,
und das Verfahren umfasst:
Antreiben (310) des Nadelhalters (21) in Reaktion auf eine erste Antriebsanweisung, um eine Nadelspitze einer Punktionsnadel (22), die am Nadelhalter (21) angebracht ist, in eine erste voreingestellte Position zu bewegen;
Erheben (320), in Reaktion auf ein Bewegungsbeendigungssignal, eines ersten Paars Stereo-Positionierungsbilder eines Bereichs relativ zur Nadelspitze der Punktionsnadel (22), Erfassen (320), auf Basis des ersten Paars Stereo-Positionierungsbilder, von Koordinaten einer realen Position der Nadelspitze und Koordinaten der ersten voreingestellten Position, und Erfassen (320) einer Koordinatendifferenz zwischen der ersten voreingestellten Position und der realen Position; und
Korrigieren (330), in Reaktion auf die Erfassung der Koordinatendifferenz, der Brustbiopsie-Positionierungsvorrichtung auf Basis der Koordinatendifferenz.

8. Verfahren nach Anspruch 7, wobei
das Erfassen von Koordinaten einer realen Position der Nadelspitze und von Koordinaten der ersten voreingestellten Position auf Basis des ersten Paars Stereo-Positionierungsbilder beinhaltet:
Identifizieren von Koordinaten einer ersten voreingestellten Markierung für jedes Bild des ersten Paars Stereo-Positionierungsbilder und Bestimmen der Koordinaten der realen Position und der Koordinaten der ersten voreingestellten Position auf Basis der Koordinaten der ersten voreingestellten Markierung, wobei jedes Bild des ersten Paars Stereo-Positionierungsbilder die erste voreingestellte Markierung einer selben Position beinhaltet; und
das Korrigieren der Brustbiopsie-Positionierungsvorrichtung auf Basis der Koordinatendifferenz beinhaltet:
Korrigieren der Brustbiopsie-Positionierungsvorrichtung auf Basis einer Koordinatendifferenz, die jedem Bild des ersten Paars Stereo-Positionierungsbilder entspricht.

9. Verfahren nach Anspruch 7 oder 8, wobei es eine Vielzahl von ersten voreingestellten Positionen gibt, und das Verfahren weiter beinhaltet:
Bewegen der Nadelspitze der Punktionsnadel (22) zu einer nächsten ersten voreingestellten Position nach Erfassen der Koordinatendifferenz an einer aktuellen ersten voreingestellten Position; und
Erfassen eines Korrekturkompensationswerts der Brustbiopsie-Positionierungsvorrichtung auf Basis von Koordinatendifferenzen, die an der Vielzahl von ersten voreingestellten Positionen erhalten wurden, und Korrigieren der Brustbiopsie-Positionierungsvorrichtung auf Basis des Korrekturkompensationswerts.

## Revendications

1. Appareil d'imagerie par rayons X du sein (100) comprenant :
un mécanisme de détection et un porte-aiguille (21), dans lequel
le mécanisme de détection inclut un tube à rayons (131) et un détecteur (132) qui est agencé à l'opposé du tube à rayons (131), une région irradiée (133) étant agencée entre le tube à rayons (131) et le détecteur (132) ; et
le porte-aiguille (21) est configuré pour se déplacer par rapport au mécanisme de détection, et le porte-aiguille (21) est agencé à l'extérieur de la région irradiée (133) ; et
un bras mécanique (30), le porte-aiguille (21) étant monté sur le bras mécanique (30), et le bras mécanique (30) étant configuré pour entraîner le porte-aiguille (21) à se déplacer par rapport au mécanisme de détection, dans lequel
le mécanisme de détection inclut en outre un portique (11), le tube à rayons (131) et le détecteur (132) étant montés sur le portique (11) ; **caractérisé en ce que**
le bras mécanique (30) est agencé indépendamment du mécanisme de détection, une position de montage du bras mécanique (30) étant relativement fixée à une position du portique (11) ; dans lequel
l'appareil d'imagerie par rayons X du sein (100) comprend en outre un hôte et une unité de photographie (40), dans lequel l'hôte est relié de manière communicative au bras mécanique (30) et configuré pour commander un mouvement du bras mécanique (30), et l'unité de photographie (40) est reliée de manière communicative à l'hôte, et le porte-aiguille (21) est agencé à l'extérieur de la région irradiée (133) sur la base d'une navigation visuelle de l'unité de photographie (40).

2. Appareil d'imagerie par rayons X du sein (100) selon la revendication 1, dans lequel
le mécanisme de détection inclut en outre une plaque de compression (141) et une plate-forme de compression (142), dans lequel la plaque de compression (141) et la plate-forme de compression (142) sont montées sur le portique (11), la plaque de compression (141) et la plate-forme de compression (142) sont agencées entre le tube à rayons (131) et le détecteur (132) et la plaque de compression (141) est agencée à l'opposé de la plate-forme de compression (142), et
l'appareil d'imagerie par rayons X du sein (100) inclut en outre une aiguille de ponction (22), dans lequel l'aiguille de ponction (22) est montée sur le porte-aiguille (21), et le bras mécanique (30) est configuré pour entraîner l'aiguille de ponction (22) à s'insérer le long d'une direction d'inclinaison par rapport à la plaque de compression (141).

3. Appareil d'imagerie par rayons X du sein (100) selon la revendication 2, dans lequel le mécanisme de détection inclut en outre un support rotatif (12), dans lequel
le support rotatif (12) est monté de manière rotative sur le portique (11), le tube à rayons (131) et le détecteur (132) sont montés sur deux extrémités d'une ouverture du support rotatif (12), et le support rotatif (12) est configuré pour tourner par rapport à la plaque de compression (141) et à la plate-forme de compression (142).

4. Appareil d'imagerie par rayons X du sein (100) selon l'une quelconque des revendications 1-3, dans lequel le bras mécanique (30) inclut au moins deux articulations de bras (31), les au moins deux articulations de bras (31) étant reliées de manière rotative, dans lequel
une extrémité d'une articulation de bras (31) des au moins deux articulations de bras (31) est relativement fixée à la position du portique (11), et le porte-aiguille (21) est monté sur une extrémité d'une autre articulation de bras (31) des au moins deux articulations de bras (31).

5. Appareil d'imagerie par rayons X du sein (100) selon la revendication 4, dans lequel au moins l'une des au moins deux articulations de bras (31) est une articulation de bras rétractable.

6. Appareil d'imagerie par rayons X du sein (100) selon la revendication 1, dans lequel l'hôte est en outre configuré pour commander un mouvement de l'unité de photographie (40), et une position de montage de l'unité de photographie (40) est relativement fixée à la position du portique (11).

7. Procédé de correction et de vérification d'un dispositif de positionnement de biopsie du sein, le dispositif de positionnement de biopsie du sein étant monté sur un appareil d'imagerie par rayons X du sein (100), dans lequel l'appareil d'imagerie par rayons X du sein (100) inclut un mécanisme de détection et un porte-aiguille (21), le mécanisme de détection incluant un tube à rayons (131) et un détecteur (132) qui est agencé à l'opposé du tube à rayons (131), une zone irradiée (133) étant agencée entre le tube à rayons (131) et le détecteur (132), le porte-aiguille (21) étant configuré pour se déplacer par rapport au mécanisme de détection, et le porte-aiguille (21) étant agencé à l'extérieur de la zone irradiée (133) ; et dans lequel l'appareil d'imagerie par rayons X du sein (100) inclut en outre un bras mécanique (30), le porte-aiguille (21) étant monté sur le bras mécanique (30), et le bras mécanique (30) étant configuré pour entraîner le porte-aiguille (21) à se déplacer par rapport au mécanisme de détection ; dans lequel le mécanisme de détection inclut en outre un portique (11), le tube à rayons (131) et le détecteur (132) étant montés sur le portique (11), **caractérisé en ce que** le bras mécanique (30) est agencé indépendamment du mécanisme de détection, une position de montage du bras mécanique (30) étant relativement fixée à une position du portique (11), et le dispositif de positionnement de biopsie du sein est relié au porte-aiguille (21), dans lequel
l'appareil d'imagerie par rayons X du sein (100) inclut en outre un hôte et une unité de photographie (40), dans lequel l'hôte est relié de manière communicative au bras mécanique (30) et configuré pour commander un mouvement du bras mécanique (30), et l'unité de photographie (40) est reliée de manière communicative à l'hôte, et le porte-aiguille (21) est agencé à l'extérieur de la région irradiée (133) sur la base d'une navigation visuelle de l'unité de photographie (40),
et le procédé comprend :
en réponse à une première instruction d'entraînement, l'entraînement (310) du porte-aiguille (21) afin de déplacer une pointe d'aiguille d'une aiguille de ponction (22) montée sur le porte-aiguille (21) vers une première position prédéfinie ;
en réponse à un signal de fin de mouvement, la collecte (320) d'une première paire d'images de positionnement stéréo d'une région par rapport à la pointe d'aiguille de l'aiguille de ponction (22), l'acquisition (320), sur la base de la première paire d'images de positionnement stéréo, de coordonnées d'une position réelle de la pointe d'aiguille et de coordonnées de la première position prédéfinie, et l'acquisition (320) d'une différence de coordonnées entre la première position prédéfinie et la position réelle ; et
en réponse à l'acquisition de la différence de coordonnées, la correction (330) du dispositif de positionnement de biopsie du sein sur la base de la différence de coordonnées.

8. Procédé selon la revendication 7, dans lequel
l'acquisition des coordonnées d'une position réelle de la pointe d'aiguille et des coordonnées de la première position prédéfinie sur la base de la première paire d'images de positionnement stéréo inclut :
pour chaque image de la première paire d'images de positionnement stéréo, l'identification de coordonnées d'une première étiquette prédéfinie, et la détermination des coordonnées de la position réelle et des coordonnées de la première position prédéfinie sur la base des coordonnées de la première étiquette prédéfinie, dans lequel chaque image de la première paire d'images de positionnement stéréo inclut la première étiquette prédéfinie d'une même position ; et
la correction du dispositif de positionnement de biopsie du sein sur la base de la différence de coordonnées inclut :
la correction du dispositif de positionnement de biopsie du sein sur la base d'une différence de coordonnées correspondant à chaque image de la première paire d'images de positionnement stéréo.

9. Procédé selon la revendication 7 ou 8, dans lequel il existe une pluralité de premières positions prédéfinies, et le procédé inclut en outre :
le déplacement de la pointe d'aiguille de l'aiguille de ponction (22) vers une première position prédéfinie suivante après avoir acquis la différence de coordonnées à une première position prédéfinie actuelle ; et
l'acquisition d'une valeur de compensation de correction du dispositif de positionnement de biopsie du sein sur la base de différences de coordonnées obtenues à la pluralité de premières positions prédéfinies, et la correction du dispositif de positionnement de biopsie du sein sur la base de la valeur de compensation de correction.
